(19) Europäisches Patentamt / European Patent Office / Office européen des brevets

(11) **EP 3 344 979 B1**

(12) **EUROPEAN PATENT SPECIFICATION**

(45) Date of publication and mention of the grant of the patent:
**27.09.2023 Bulletin 2023/39**

(21) Application number: **16750741.7**

(22) Date of filing: **04.08.2016**

(51) International Patent Classification (IPC):
**G01N 23/04** (2018.01)     **A61B 6/00** (2006.01)
**G21K 1/00** (2006.01)

(52) Cooperative Patent Classification (CPC):
**G01N 23/046; A61B 6/484;** G21K 1/06;
G21K 2207/005

(86) International application number:
**PCT/EP2016/068621**

(87) International publication number:
**WO 2017/036726 (09.03.2017 Gazette 2017/10)**

(54) **DUAL PHASE GRATING INTERFEROMETER FOR X-RAY PHASE CONTRAST IMAGING**

DOPPEL-PHASENGITTERINTERFEROMETER ZUR PHASENKONTRAST-RÖNTGENBILDGEBUNG

INTERFÉROMÈTRE À DOUBLE RÉSEAU DE PHASE POUR IMAGERIE À CONTRASTE DE PHASE AUX RAYONS X

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.09.2015 EP 15183946**

(43) Date of publication of application:
**11.07.2018 Bulletin 2018/28**

(73) Proprietor: **Paul Scherrer Institut**
**5232 Villigen PSI (CH)**

(72) Inventors:
• **KAGIAS, Matias**
**8002 Zürich (CH)**
• **STAMPANONI, Marco**
**5304 Endingen (CH)**
• **WANG, Zhentian**
**5200 Brugg (CH)**

(74) Representative: **Fischer, Michael**
**Siemens AG**
**Postfach 22 16 34**
**80506 München (DE)**

(56) References cited:
**EP-A1- 1 447 046**     **EP-A1- 1 731 099**
**WO-A1-2014/194995**     **US-A1- 2015 036 795**

**Description**

The present invention relates to an arrangement for x-rays, in particular hard x-rays, for obtaining quantitative x-ray images from a sample.

Grating interferometry (GI) constitutes a very promising technique for commercial X-ray phase-contrast applications, since it works with traditional X-ray tubes, is mechanically robust and has modest requirements for mono-chromaticity and spatial coherence, for instance, invention described in Ref.[1] could relax the coherence requirement. System described in Ref.[2] with static grating-detector configuration further simplifies the acquisition protocol of GI, paving the road for its commercial applications. In the last few years, several exciting applications of this technique have been reported, ranging from material inspection to medical imaging. To carry out the transition of grating interferometry from the synchrotrons and physics laboratory to the commercial setting (medical, homeland security, non-destructive testing or similar), the technology has to be further developed to cover a large field of view (FOV), to allow operation in a broad energy range and to be dose efficient. Both US 2015/036795 and WO 2014/194995 disclose apparatus for x-ray phase contrast imaging.

These issues quickly became serious problems, as in all the suggestions presented so far either absorption gratings of very large aspect ratio were needed or the usage of high-resolution detectors was required -- which are notoriously incompatible with large areas -- or the costs in terms of additional radiation deposited in the sample due to the absorption gratings were inacceptable. Although the FOV issue can be partially mitigated using a slit-scanning approach, the requirement on high aspect ratio absorption grating for high-energy applications remains a huge fabrication challenge. An even more critical and intrinsic issue of grating interferometry is the dose efficiency due to the usage of absorption grating (the so-called analyzer grating that is in front of the detector). Though the absorption grating decouples the system sensitivity from the system spatial resolution which in fact is the key to the success of the grating interferometer, its existence reduces the system's dose efficiency and flux efficiency by half which severely limits the applications of grating interferometry, especially in medical imaging.

An interferometric method without the absorption grating will naturally solve the dose and flux efficiency issue, and avoid the fabrication challenge, ideally with fast data acquisition, will become desirable for clinical and industrial applications.

The present invention provides an arrangement for x-rays, in particular hard x-rays, for obtaining quantitative x-ray images from a sample, as set forth in claim 1.

The present invention represents a crucial step towards X-ray absorption-grating-less interferometry. The present invention generates a large period interference pattern (i.e. removing the need of a high-resolution detector) by using only phase gratings. One phase grating is used to create a secondary source, and another phase grating is to form an inference pattern under the illumination of the secondary source. This new phase-phase gratings solution instead of the phase-absorption or absorption-absorption suggestions will allow to exploit essentially 100% of the photons (as the attenuation in the phase gratings can be considered as negligible) therefore solving the limitation of all interferometers presented so far, which are cutting half of the photons (or more) after the sample. In addition, and probably the most striking point of this invention, since the phase shift varies linearly with the energy (in comparison to the $E^{-3}$ dependency of the attenuation signal) it results that phase-shifting gratings for high energies > 60 keV are easily achievable with state-of-the-art fabrication methods. In general, the fabrication of a phase grating is much easier than absorption grating, and the quality control can be done better as well. These points remove the most significant burden from a wide application of grating interferometry.

The present invention may have preferred embodiments which can be generated by one or a suitable combination of some or all the features listed below:

- the phase-shift gratings G1 and G2 are both $\pi$ shift gratings, or both $\pi/2$ shift gratings, or one of them is $\pi$ shift grating and the other is $\pi/2$ shift grating;

- the phase-shift grating is made by deep etching into silicon, a polymer or similar material, preferable for low energy X-ray photons; or deposit heavy metal into gaps of low-absorbing structure or grow heavy metal on low-absorbing substrate and as use the metal as the phase shift material, preferably for high energy X-ray photons;

- in the embodiments of the invention, the first grating G1 creates a periodic interference pattern with pitch piat a known distance (Talbot effect) downstream and acts as structured illumination onto the second grating G2, which further creates a periodic interference pattern with pitch $p'_2$ at the detector plane. $p'_1$ and $p'_2$ match the radius of curvature of an incident wavefront by the relation $p'_1 = \frac{1}{\eta} p_1 \frac{d_1 + l_1}{l_1}$, $p'_2 = \frac{1}{\eta} p_2 \frac{d_2 + l_2}{l_2}$ and $\frac{p'_1}{p'_2} = \frac{d_2}{l_2}$, where $p_1$ and $p_2$ are the pitch of G1 and G2, respectively, $l_1$ is the distance between the source (or G0 if G0 is used) to G1 distance, $d_1$ is the distance between G1 and the created structured illumination, $d_2$ is the distance between the structured illumination and G2, $l_2$ is the distance between G2 and the detector, $\eta = 1$ for $\pi$ shift grating while $\eta = 2$ for $\pi/2$ shift grating;

- the phase shift of G1 and G2 and the distances between the source (or G0 if G0 is used), G1, G2 and the detector are adapted to a photon energy corresponding to an emission line of the X-ray generator used as the source;

- a mechanism is comprised to vary the angular orientation, around the optical axis, of the G1 and G2 with respect to each other, so that the orientation of the interference pattern on the detector plane is tuned in order to obtain sample information along a specific direction;

- a mechanism is comprised to vary the distance between G1 and G2, or that between source (or G0 if G0 is used) and G1, or that between G2 and the detector, along the optical axis, so that the pitch of the interference pattern on the detector plane is tuned in order to obtain sample image with desired spatial resolution, or desired sensitivity, or work with detectors with certain specific pixel size;

- a mechanism is comprised to vary the distance between G1 and G2, or that between source (or G0 if G0 is used) and G1, or that between G2 and the detector, along the optical axis, so that the duty cycle of the interference pattern on the detector plane is tuned in order to obtain interference pattern with specific duty cycle;

- a mechanism is comprised to incline the phase grating G1 and G2 together or with respect to each other along the optical axis, so that the phase shifts of G1 and G2 is tuned to be $\pi$ shift or $\pi/2$ shift to match a tunable photon energy therefore the arrangement can work at different photon energy with the same phase shift gratings;

- the phase gratings G1 and G2 are identical, featuring a symmetric system design, that is to be said, $l_1 = l_2$ and $d_1 = d_2$;

- the phase gratings G1 and G2 have different pitches, featuring an asymmetric system design;

- G0 is not used but the X-ray source comprises 1D or 2D array of individual sources that may be mutually incoherent and whose lateral separation

$$p_{0=}p_1' \times \frac{l_1}{d_1}$$ or integer multiples thereof;

- the array of X-ray source is generated by using an anode that structured topographically or assembled in a mosaic manner from the same or different materials;

- a mechanism is comprised to place a sample (S) to be investigated between the X-ray source (or G0 if G0 is used) and G1, or between G1 and G2, or between G2 and the detector;

- an analysis procedure is implemented for obtain the absorption, differential phase contrast and scattering contrast of the sample that comprises the steps of recording two intensity images of the interference pattern (with sample and without sample) on the detector, and analyzing the local distortions of the two interference patterns by either spatial Fourier analysis or spatial correlation analysis, and then retaining the three contrast of the sample;

- a mechanism is comprised to lateral move one of the gratings (G0, G1 and G2) with a fraction of its pitch and records a serial of images on the detector, and an analysis procedure is implemented for this phase-stepping data scan that comprises the steps of calculating, for each element of the detector, the Fourier transform of the intensity curve measured in the element, and then retaining the Fourier components as signals for further processing;

- means for rotating the sample relatively to the remaining components to perform data collection for a tomographic scan.

- the phase grating G1 and G2 are fabricated on the two sides of the same wafer, with grating structures either parallel to each other or with a predefined angle;

- G1 is a 2D chessboard/mesh-type grating, and G2 is a 2D array grating, with each element of the array to be a circular grating; and

- G1 and G2 are absorption gratings.

Preferred embodiments of the present invention are described hereinafter in detail with reference to the attached drawings which depict in:

Fig. 1    schematically a conventional Talbot-Lau grating interferometer;

Fig. 2    a sketch of the dual phase gratings interferometer and its working mechanism; (a) 3D sketch of the proposed interferometer; (b) Top view of the proposed interferometer; (c) Side view of the proposed interferometer;

Fig. 3.    experimental results of the proposed interferometer;

Fig. 4    fringe tuning by changing the inter-grating (G1-G2) distance;

Fig. 5     imaging a plastic tube using the arrangement shown in Fig. 2; (a) The transmission image; (b) The differential phase image;

Fig. 6     tilting G1 and G2 together along the optical axis to work with different design energy; and

Fig. 7     schematically an example of the tunable length scale sensitivity for an apparatus according to Figure 1.

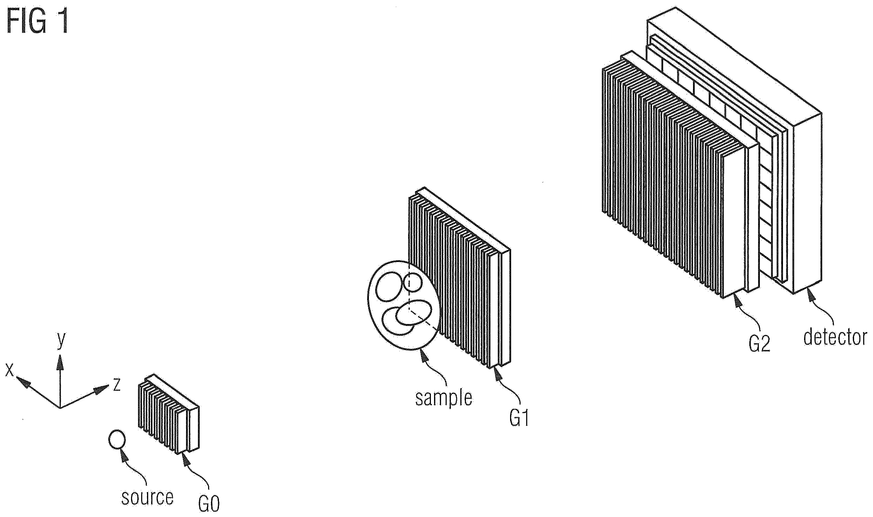

A conventional Talbot-Lau grating interferometer is shown in Fig. 1, where an X-ray source emits an X-ray beam along an X-ray beam path here in the z-direction. The X-ray beam passes consequently through an absorption grating G0, a probe, a first phase-shift grating G1 and a second phase shift grating G2. The use of the absorption grating G0 is optional, depending on the spatial coherence properties of the X-ray source. The first and second phase-shift grating comprise grating line that extend in y-direction.

The conventional grating interferometer decouples the system sensitivity and the detector spatial resolution by introducing an acquisition protocol named phase stepping. In the phase stepping procedure, one of the gratings G1, G2 is laterally translated step-by-step, perpendicular to the grating lines, and a series of images are taken for each step. For each element of the detector, a sinusoid intensity curve is then recorded. The absorption contrast (AC), differential phase contrast (DPC) and scattering contrast (SC) of the sample are calculated by comparing the intensity curve without the sample and that with the sample. Defining for each pixel on the detector the mean, phase and visibility of the intensity curve with sample as $I_s, \phi_s, V_s$, and without sample as $I_b, \phi_b, V_b$, yields:

$$AC = -\log\left(\frac{I_s}{I_b}\right)$$

$$(1)$$

$$DPC = \phi_s - \phi_b$$

$$(2)$$

$$SC = -log\left(\frac{V_s}{V_b}\right)$$

$$(3)$$

One disadvantage of the conventional grating interferometer is that the phase stepping procedure is quite time-consuming and therefore renders applications that need fast scanning or time-resolution not possible. Furthermore, it requires additional mechanical movement of the optical components, which poses higher requirements on the system stability and design. Both issues are undesired for clinical and industrial applications.

To avoid the time-consuming phase stepping scan, a few single-shot X-ray phase-contrast imaging (PCI) methods using gratings have been proposed. Like conventional X-ray imaging modalities, single-shot PCI methods only take two images: one reference image without sample and one sample image. In order to obtain three different contrasts in a single shot, these methods require that the detector has sufficient resolution to resolve the fringe pattern generated by the gratings, via either interference effect or simple projection, directly. The sensitivity of such methods is largely inverse proportional to the pitch of the fringe on the detector, therefore is indirectly limited by the detector pixel size.

Nevertheless, the existing single-shot methods still use absorption gratings to generate the fringe pattern, either use it together with a phase grating to create a large period Moiré fringe, or use large pitch absorption grating or grid to create a projected fringe directly.

One possible solution without using an absorption grating is the inverse geometry grating interferometer, however this method is dictated by the discrete property of the Talbot(-Lau) effect, that being said, the system total length, the pitches of the gratings and the photon energy are strongly coupled and can't be tuned continuously and arbitrarily. For the energy range (8-30 keV) used in literatures, the inverse geometry interferometer results in either impractical, very long system length (>> 2m) or very small pitch G0 (<< 2$\mu$m) that is not possible to fabricate at this stage. For clinical and industrial applications, the system length has to be within a reasonable range to utilize the limited flux of the X-ray tube (since the flux is inverse proportional to the $R^2$, as $R$ to be the source to detector distance). A compact system is usually desired due to the higher flux efficiency, therefore the inverse geometry approach is not practical.

Alternative single-shot DPC methods using speckles have also been proposed. However these methods pose harsh requirements on the coherence of the X-ray source, therefore are limited to either synchrotrons or micro-focal X-ray source with very long setup (3m).

The present invention is essentially a single-shot DPC method, overcoming the aforementioned limitations of existing methods. The present invention consists of an X-ray arrangement, which measures absorption, phase and scattering signals from a sample with the usage of a phase-phase grating combination (G1 and G2 are both phase-shift gratings). It addresses the following difficulties:

- It exploits all photons transmitted through the sample to generate contrast;
- It can be implemented at energies varying over a very broad range;
- It has a tunable working energy;
- It can cover large field of view;
- It can feasibly change length scale sensitivity;

- It works in symmetrical, asymmetrical and magnified geometry; and
- Is compatible with single-shot imaging.

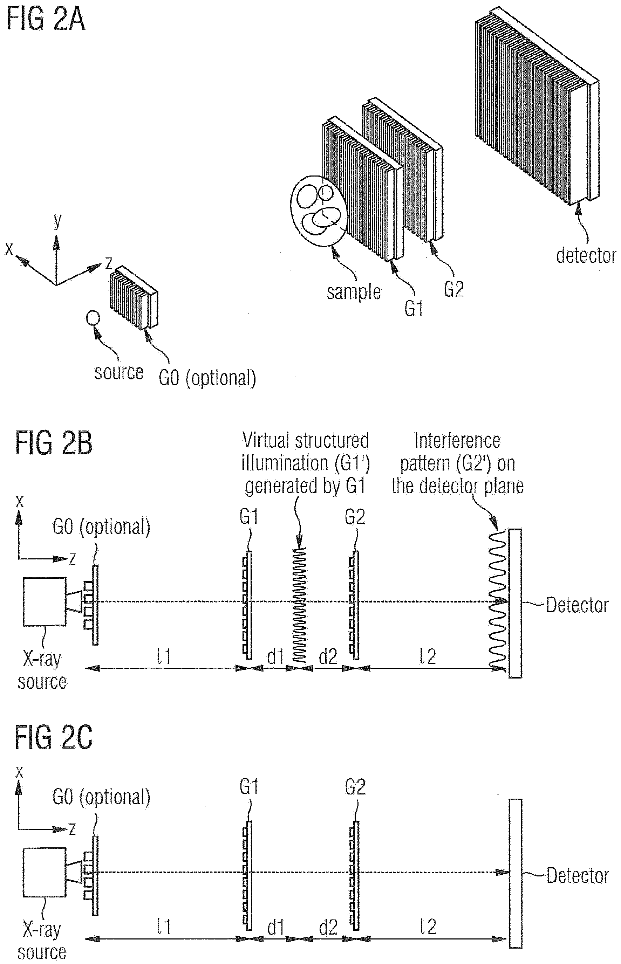

The idea behind the present invention, dubbed as dual phase gratings interferometer, is shown in Fig. 2. Since only phase gratings G1 and G2 are used, the dose and flux efficiency of the present invention is much better than existing methods. Fig. 2 shows a sketch of the dual phase gratings interferometer and its working mechanism; (a) 3D sketch of the proposed interferometer; (b) Top view of the proposed interferometer; (c) Side view of the proposed interferometer.

In the proposed invention, a first phase grating G1 creates a periodic interference pattern at a known distance downstream. This intensity distribution can be considered as a secondary source with structured illumination, which is used to generate a large-period interference pattern on the detector with the usage of a second phase grating G2. In principle, the optional absorption grating G0, the first phase-shift grating G1 and the virtual fringe G1' constitute a conventional Talbot (-Lau) interferometer, and the virtual fringe G1', the second phase-shift grating G2 and the interference pattern G2' on the detector constitute another Talbot (-Lau) interferometer. Depending on the pitches of the gratings, such a device can be "symmetric" (i.e. G1 and G2 are identical for fabrication benefit, resulting in equal distance between the source-to-G1 and the G2-to-detector) or asymmetrical (i. e. arbitrary configuration for flexible system design).

Like conventional Talbot-(Lau) grating interferometer, G0 is only necessary when an extended X-ray source is used in order to produce sufficient coherence. However, unlike an inverse geometry grating interferometer whose G0 may have much smaller pitch than G1, the pitch of G0 in the proposed interferometer is always equal to or larger than the pitch of G1, therefore won't pose any fabrication difficulty.

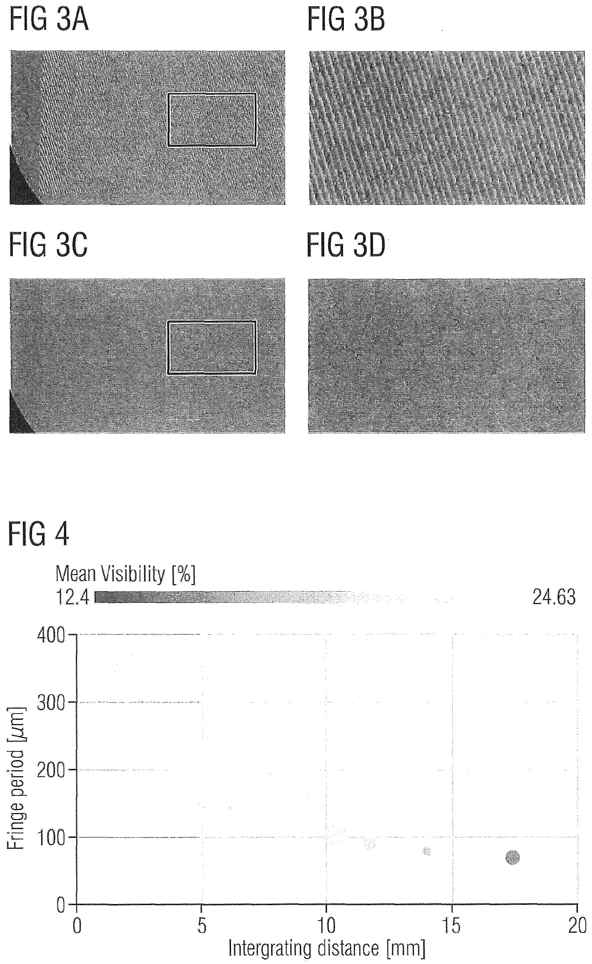

An example of the interference fringes generated with the interferometer proposed in Fig. 2 is shown in Fig. 3. The experiment was carried on with a micro-focal X-ray tube (operated at 50kVp) and two identical phase gratings ($\pi$ shift @ 17KeV) with the pitch of 1.2$\mu$m. The interference pattern on the detector plane only exists when the second phase-shift grating G2 is introduced.

Fig. 3 shows the experimental results of the proposed interferometer. (a) and (c) are the images acquired on the detector with and without the second phase-shift grating G2, respectively. Once the second phase-shift grating G2 is removed from the beam path, the interference pattern disappeared. (b) and (d) are the corresponding ROI images of the rectangular in (a) and (c).

The proposed invention explores the same physical effect (i.e. Talbot effect) as the conventional Talbot-(Lau) grating interferometer, therefore it inherits some similar properties, for instance, the relationship between the pitches of the gratings and the distances ($l_1$ and $d_1$, $l_2$ and $d_2$) are following the same formulas, but due to the unique design, the proposed approach provides much more flexibility in designing a compact system.

For example, consider a single-shot DPC system that requires a design energy of 25KeV and a total system length of 1m, and has a detector that can resolve interference pattern with 100$\mu$m pitch. An inverse geometry system will require an absorption grating G0 with less than 1$\mu$m pitch and the G0-G1 distance will be less than 1cm which results in useless FOV. Using the proposed dual phase grating approach with a symmetric design, the pitch of the optional G0 is 100$\mu$m and the pitches of the two phase gratings will be 1$\mu$m. Compared to the inverse geometry case, it's obviously much easier to fabricate 1$\mu$m phase grating than absorption grating.

The proposed invention works well with monochromatic beam, however when working with polychromatic beam, it provides more flexibilities to cope with different requirements due to the broad energy spectrum acceptance of the Talbot-(Lau) interferometer. In one implementation, the distance between G1 and G2 can be tuned, resulting in variable pitch of interference fringe on the detector. This feature can be used to tune the system's sensitivity or to match a specific detector pixel size, for instance, using different binning of the detector. An experimental result is shown in Fig. 4.

Fig. 4 shows a fringe tuning by changing the inter-grating (G1-G2) distance. The designed distance between the first phase-shift grating G1 and the second phase-shift grating G2 distance is 5mm in this case. Highest visibility is obtained at the designed position. While tuning the inter-grating distance, the pitch of the fringe is tuned as well.

The orientation of the interference fringe on the detector can be easily changed by rotating one of G1 and G2, or G1 and G2 together. In the meantime, the pitch of the fringe will stay constant. This allows one to obtain multiple directional DPC and scattering signals of the sample with constant sensitivity.

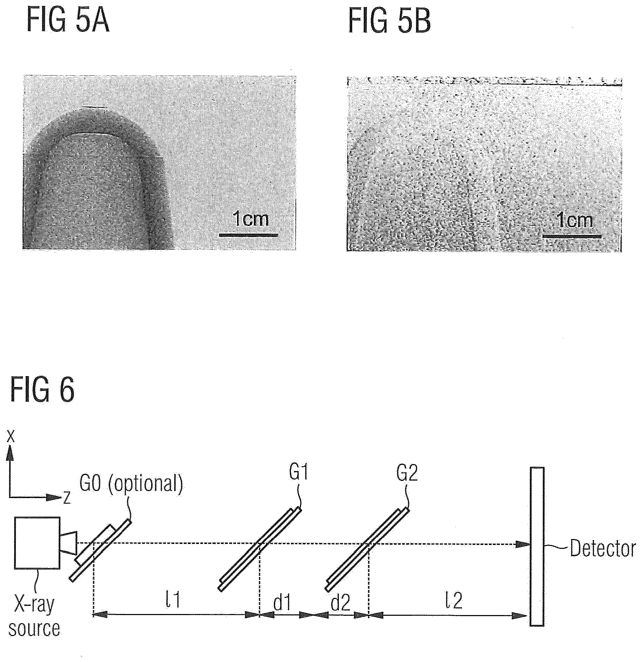

The sample can be placed between the X-ray source (or G0 if G0 is used) and G1, or between G1 and G2, or between G2 and the detector. To achieve the highest sensitivity, it's preferable to place the sample close to G1 or G2. An imaging result is shown in Fig. 5. In detail, Fig. 5 shows an imaging a plastic tube using the proposed method. Figure 5(a) represents the transmission image; Figure 5(b) the differential phase image accordingly.

For the proposed interferometer, the inter-grating distance (G1-G2) can be very small. Eventually they can be fabricated on the two sides of the same wafer (i.e. etching on both sides of a Silicon wafer), therefore G1 and G2 become one optical component. This will significantly reduce the complexity of the setup.

Due to the nature of phase grating, the proposed interferometer can be easily tuned to work perfectly with different design energies. By tilting G1 and G2 together along the optical axis (see Fig. 6), the effective height of the phase-shifting structure is increased, and the system naturally works with higher design energy. Fig. 6 shows the tilting of G1 and G2 together along the optical axis to work with different design energy.

Another advantage of using purely phase gratings is that it's easier to work with high-energy photons. High Z material (i.e. Au) can be used as phase shift material in high-energy situations. The required aspect ratio of the grating is much lower for phase grating compared to absorption grating, resulting in significant fabrication benefits. For example, for 100KeV photon, absorption grating with 10% transmission will require of Gold structure of at least 230$\mu$m, while a $\pi$-shift Gold grating will only need 19.8$\mu$m.

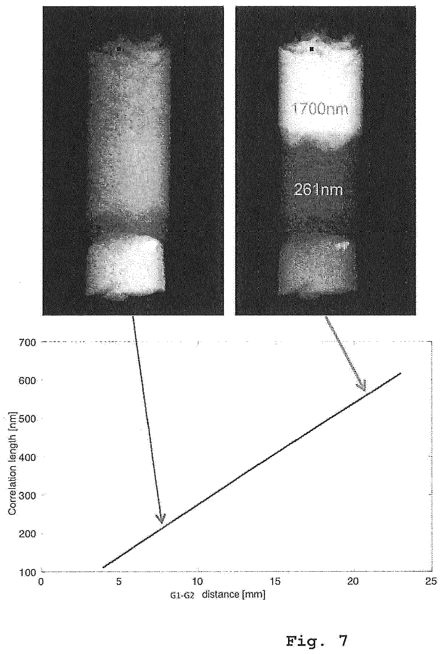

Figure 7 schematically shows an example of the tunable length scale sensitivity for an apparatus according to Figure 1. The samples are two layers of Silica spheres with the diameter of 1700 nm and 261 nm, respectively. By simply tuning the distance between G1 and G2, the system is sensitive to sphere diameter between 100nm up to 600 nm, and the selectivity can be clearly seen from the different contrasts in the dark-field images. The effective pixel size is about 12 $\mu$m, which is far bigger than the sphere diameter. The bottom figure shows the calculated system length scale sensitivity changed with the G1-G2 distance.

Reference

[1] C. David, European patent, EP 1447046A1 (2004-08-18)
[2] E. Roessl and T. Koehler, US patent, US 2015/036795A1 (2015-02-05)

Claims

1. An arrangement for x-rays, in particular hard x-rays, for obtaining quantitative x-ray images from a sample, said arrangement being **characterized by**:

   a) an X-ray source (x-ray) providing an X-ray beam along an X-ray beam path;
   b1) a first phase-shift grating (G1) and a second phase-shift grating (G2) placed consecutively along the X-ray beam path, wherein the first and the second phase-shift grating (G1, G2) each comprising a periodic grating structure generating a X-ray phase shift difference,
   b2) the second phase-shift grating (G2) either of $\pi$ or odd multiples thereof (denoted as $\pi$ shift), or $\pi/2$ or $\pi/2+N\times2\times\pi$ (denoted as $\pi/2$ shift), where N is a integer number; and wherein
   b3) the first phase-shift grating (G1) is adapted to create a periodic interference pattern at a known distance downstream, such that said periodic interference pattern acts as a structured illumination onto the second phase-shift grating (G2) to generate a large-period interference pattern on a position-sensitive detector (PSD);
   c) said position-sensitive detector (PSD) with spatially modulated detection sensitivity having a number of individual pixels;
   d) means for recording the images of the detector (PSD);
   e) means for evaluating the intensities for each pixel in a series of images in order to identify the characteristic of the object for each individual pixel as an absorption dominated pixel and/or a differential phase contrast dominated pixel and/or an x-ray scattering dominated pixel.

2. The arrangement according to claim 1, wherein an absorption grating (G0) is placed in front of, or embedded into the X-ray source.

3. The arrangement according to claim 1 or 2, wherein the phase-shift gratings (G1, G2) are line (1D) or 2D array (i.e. chessboard) phase gratings.

4. The arrangement according to any of the preceding claims,
   **characterized in that**
   the angular orientation of the two phase-shift gratings (G1, G2) is varied relatively to each other around the optical axis, to tune the orientation of the interference pattern on the detector plane in order to obtain sample information along a specific direction.

5. The arrangement according to any of the preceding claims,
   **characterized in that**
   the distance between the two phase-shift gratings (G1, G2), or the distance between the source or the absorption grating (G0) if the absorption grating (G0) is used and the first phase shift grating (G1), or the distance between the second phase-shift grating (G2) and the detector, along the optical axis, is changed, to tune the pitch of the interference pattern on the detector plane in order to obtain sample image with desired spatial resolution, or desired sensitivity, or work with detectors with certain specific pixel size.

6. The arrangement according to any of the preceding claims,
   **characterized in that**
   the distance between the two phase-shift gratings (G1, G2), or the distance between the source or the absorption grating (G0) if the absorption grating (G0) is used and the first phase shift grating (G1), or the distance between the second phase-shift grating (G2) and the detector, along the optical axis, is changed, to tune the duty cycle of the interference pattern on the detector plane in order to obtain interference pattern with specific duty cycle.

7. An arrangement according to any of the preceding claims, **characterized in that** the phase-shift gratings (G1, G2) are inclined together or with respect to each other along the optical axis to tune the phase shifts of the phase-shift gratings (G1, G2) to be $\pi$ shift or $\pi/2$ shift in order to match a tunable photon energy allowing the arrangement to work at different photon energy with the same phase-shift gratings.

8. The arrangement according to any of the preceding claims, wherein the sample (S) to be investigated is placed between the X-ray source (or the absorption grating (G0) if the absorption grating (G0) is used and the first phase-shift grating (G1), or between the first phase shift grating (G1) and the second phase-shift grating (G2), or between the second phase-shift grating (G2) and the detector.

9. The arrangement according to any of the preceding claims, **characterized in that** one of the gratings (G0, G1 and G2) is laterally moved with a fraction of its pitch and records a serial of images on the detector in terms of the phase-stepping scan, and an analysis procedure is implemented for the data of this phase-stepping scan that comprises the steps of calculating, for each pixel of the detector, the Fourier transform of the intensity curve measured in the pixel, and then retaining the Fourier components as signals for further processing.

10. The arrangement according to any of the preceding claims, wherein the phase-shift grating (G1, G2) are fabricated on the two sides of a wafer, with grating structures either parallel to each other or with a predefined angle.

11. The arrangement according to any of the preceding claims, wherein the first phase-shift grating (G1) is a 2D chessboard/mesh-type grating, and the second grating (G2) is a 2D array grating, with each element of the array to be a circular grating.

12. The arrangement according to any of the preceding claims, wherein the two phase-shift gratings (G1, G2) are absorption gratings.

**Patentansprüche**

1. Anordnung für Röntgenstrahlung, insbesondere harte Röntgenstrahlung, zum Erlangen quantitativer Röntgenbilder von einer Probe, wobei die Anordnung durch Folgendes gekennzeichnet ist:

   a) eine Röntgenquelle (Röntgenstrahl), die entlang eines Röntgenstrahlengangs einen Röntgenstrahl bereitstellt;

   b1) ein erstes Phasenverschiebungsgitter (G1) und ein zweites Phasenverschiebungsgitter (G2), die nacheinander entlang des Röntgenstrahlengangs platziert sind, wobei das erste und das zweite Phasenverschiebungsgitter (G1, G2) jeweils eine periodische Gitterstruktur umfassen, die eine Röntgenphasenverschiebungsdifferenz generiert,

   b2) das zweite Phasenverschiebungsgitter (G2) entweder von $\pi$ oder einem ungeraden Vielfachen davon (als n-Verschiebung bezeichnet) oder $\pi/2$ oder $\pi/2+N\times2\times\pi$ (als n/2-Verschiebung bezeichnet), wobei N eine ganze Zahl ist; und wobei

   b3) das erste Phasenverschiebungsgitter (G1) angepasst ist, um in einem bekannten Abstand stromabwärts derart ein periodisches Interferenzmuster zu erzeugen, dass das periodische Interferenzmuster als eine strukturierte Beleuchtung auf das zweite Phasenverschiebungsgitter (G2) einwirkt, um ein Interferenzmuster mit großer Periode auf einem positionsempfindlichen Detektor (PSD) zu generieren;

   c) der positionsempfindliche Detektor (PSD) mit räumlich modulierter Detektionsempfindlichkeit, der eine Anzahl an einzelnen Pixeln aufweist;

   d) ein Mittel zum Aufzeichnen der Bilder des Detektors (PSD);

   e) ein Mittel zum Auswerten der Intensitäten für jedes Pixel in einer Reihe von Bildern, um das Merkmal des Objekts für jedes einzelne Pixel als ein durch Absorption dominiertes Pixel und/oder ein durch differentiellen Phasenkontrast dominiertes Pixel und/oder ein durch Röntgenstreuung dominiertes Pixel zu identifizieren.

2. Anordnung nach Anspruch 1, wobei ein Absorptionsgitter (G0) der Röntgenquelle vorgeschaltet oder in diese eingebettet ist.

3. Anordnung nach Anspruch 1 oder 2, wobei die Phasenverschiebungsgitter (G1, G2) Linien- (1D-) oder 2D-Array-Phasengitter (z. B. Schachbrett) sind.

4. Anordnung nach einem der vorhergehenden Ansprüche, **dadurch gekennzeichnet, dass** die Winkelausrichtung der zwei Phasenverschiebungsgitter (G1, G2) relativ zueinander um die optische Achse variiert wird, um die Ausrichtung des Interferenzmusters auf der Detektorebene abzustimmen, um Probeninformationen entlang einer spezifischen Richtung zu erlangen.

5. Anordnung nach einem der vorhergehenden Ansprüche, **dadurch gekennzeichnet, dass**

der Abstand zwischen den zwei Phasenverschiebungsgittern (G1, G2) oder der Abstand zwischen der Quelle oder dem Absorptionsgitter (G0), wenn das Absorptionsgitter (G0) verwendet wird, und dem ersten Phasenverschiebungsgitter (G1) oder der Abstand zwischen dem zweiten Phasenverschiebungsgitter (G2) und dem Detektor entlang der optischen Achse geändert wird, um das Maß des Interferenzmusters auf der Detektorebene abzustimmen, um ein Probenbild mit einer gewünschten räumlichen Auflösung oder einer gewünschten Empfindlichkeit zu erlangen oder mit Detektoren mit einer bestimmten Pixelgröße zu arbeiten.

6. Anordnung nach einem der vorhergehenden Ansprüche, **dadurch gekennzeichnet, dass** der Abstand zwischen den zwei Phasenverschiebungsgittern (G1, G2) oder der Abstand zwischen der Quelle oder dem Absorptionsgitter (G0), wenn das Absorptionsgitter (G0) verwendet wird, und dem ersten Phasenverschiebungsgitter (G1) oder der Abstand zwischen dem zweiten Phasenverschiebungsgitter (G2) und dem Detektor entlang der optischen Achse geändert wird, um das Tastverhältnis des Interferenzmusters auf der Detektorebene abzustimmen, um ein Interferenzmuster mit einem spezifischen Tastverhältnis zu erlangen.

7. Anordnung nach einem der vorhergehenden Ansprüche, **dadurch gekennzeichnet, dass** die Phasenverschiebungsgitter (G1, G2) zusammen oder in Bezug zueinander entlang der optischen Achse geneigt sind, um die Phasenverschiebungen der Phasenverschiebungsgitter (G1, G2) auf eine n-Verschiebung oder eine n/2-Verschiebung abzustimmen, um zu einer abstimmbaren Photonenenergie zu passen, die es der Anordnung ermöglicht, bei unterschiedlicher Photonenenergie mit denselben Phasenverschiebungsgittern zu arbeiten.

8. Anordnung nach einem der vorhergehenden Ansprüche, wobei die zu untersuchende Probe (S) zwischen der Röntgenquelle (oder dem Absorptionsgitter (G0), wenn das Absorptionsgitter (G0) verwendet wird, und dem ersten Phasenverschiebungsgitter (G1) oder zwischen dem ersten Phasenverschiebungsgitter (G1) und dem zweiten Phasenverschiebungsgitter (G2) oder zwischen dem zweiten Phasenverschiebungsgitter (G2) und dem Detektor platziert ist.

9. Anordnung nach einem der vorhergehenden Ansprüche, **dadurch gekennzeichnet, dass** eines der Gitter (G0, G1 und G2) um einen Bruchteil seines Maßes seitlich bewegt wird und eine Reihe von Bildern auf dem Detektor im Hinblick auf den phasenschrittweisen Scan aufzeichnet und ein Analyseverfahren für die Daten dieses phasenschrittweisen Scans implementiert wird, der die Schritte des Berechnens der Fourier-Transformation der in dem Pixel gemessenen Intensitätskurve für jedes Pixel des Detektors und danach des Beibehaltens der Fourier-Komponenten als Signale für eine weitere Verarbeitung umfasst.

10. Anordnung nach einem der vorhergehenden Ansprüche, wobei die Phasenverschiebungsgitter (G1, G2) auf den zwei Seiten eines Wafers hergestellt sind, wobei die Gitterstrukturen entweder parallel zueinander oder in einem vordefinierten Winkel verlaufen.

11. Anordnung nach einem der vorhergehenden Ansprüche, wobei das erste Phasenverschiebungsgitter (G1) ein 2D-Schachbrett-/Maschengitter ist und das zweite Gitter (G2) ein 2D-Array-Gitter ist, wobei jedes Element des Arrays ein kreisförmiges Gitter sein muss.

12. Anordnung nach einem der vorhergehenden Ansprüche, wobei die zwei Phasenverschiebungsgitter (G1, G2) Absorptionsgitter sind.

**Revendications**

1. Agencement pour rayons X, en particulier rayons X durs, destiné à obtenir des images quantitatives par rayons X à partir d'un échantillon, ledit agencement étant **caractérisé par** :

    a) une source de rayons X (rayon X) fournissant un faisceau de rayons X le long d'un trajet de faisceau de rayons X ;
    b1) un premier réseau de diffraction à déphasage (G1) et un second réseau de diffraction à déphasage (G2) placés consécutivement le long du trajet de faisceau de rayons X, le premier et le second réseau de diffraction à déphasage (G1, G2) comprenant chacun une structure de réseau de diffraction périodique générant une différence de déphasage de rayons X,
    b2) le second réseau de diffraction à déphasage (G2) soit de $\pi$ ou de multiples impairs de celui-ci (indiqué par déphasage $\pi$), soit de $\pi/2$ soit $\pi/2$ + N $\times$ 2 $\times$ $\pi$ (indiqué par déphasage $\pi/2$), où N est un nombre entier ; et
    b3) le premier réseau de diffraction à déphasage (G1) étant adapté pour créer un motif d'interférence périodique à un aval de distance connue, de sorte que ledit motif d'interférence périodique agit comme un éclairage structuré sur le second réseau de diffraction à déphasage (G2) afin de générer un motif d'interférence à grande période sur un détecteur (PSD) sensible à la position ;
    c) ledit détecteur (PSD) sensible à la position ayant une sensibilité de détection modulée spatialement ayant un nombre de pixels individuels ;

d) un moyen d'enregistrement des images du détecteur (PSD) ;

e) un moyen d'évaluation des intensités pour chaque pixel dans une série d'images afin d'identifier la caractéristique de l'objet pour chaque pixel individuel sous la forme d'un pixel dominé par absorption et/ou d'un pixel dominé par contraste de phase différentielle et/ou d'un pixel dominé par diffusion des rayons X.

2. Agencement selon la revendication 1, un réseau de diffraction par absorption (G0) étant placé à l'avant, ou enchâssé dans la source de rayons X.

3. Agencement selon la revendication 1 ou 2, les réseaux de diffraction à déphasage (G1, G2) étant des réseaux de diffraction à phase linéaire (1D) ou à réseau 2D (c'est-à-dire en damier).

4. Agencement selon l'une quelconque des revendications précédentes, **caractérisé en ce que** l'orientation angulaire des deux réseaux de diffraction à déphasage (G1, G2) varie relativement l'une par rapport à l'autre autour de l'axe optique, pour ajuster l'orientation du motif d'interférence sur le plan du détecteur afin d'obtenir des informations d'échantillon le long d'un sens spécifique.

5. Agencement selon l'une quelconque des revendications précédentes, **caractérisé en ce que** la distance entre les deux réseaux de diffraction à déphasage (G1, G2), ou la distance entre la source ou le réseau de diffraction par absorption (G0) si le réseau de diffraction par absorption (G0) est utilisé et le premier réseau de diffraction à déphasage (G1), ou la distance entre le second réseau de diffraction à déphasage (G2) et le détecteur, le long de l'axe optique, est modifiée, pour ajuster le pas du motif d'interférence sur le plan du détecteur afin d'obtenir une image d'échantillon ayant une résolution spatiale souhaitée, ou une sensibilité souhaitée, ou fonctionne avec des détecteurs ayant une certaine taille de pixel spécifique.

6. Agencement selon l'une quelconque des revendications précédentes, **caractérisé en ce que** la distance entre les deux réseaux de diffraction à déphasage (G1, G2), ou la distance entre la source ou le réseau de diffraction par absorption (G0) si le réseau de diffraction par absorption (G0) est utilisé et le premier réseau de diffraction à déphasage (G1), ou la distance entre le second réseau de diffraction à déphasage (G2) et le détecteur, le long de l'axe optique, est modifiée, pour ajuster le cycle de charge du motif d'interférence sur le plan du détecteur afin d'obtenir un motif d'interférence ayant un cycle de charge spécifique.

7. Agencement selon l'une quelconque des revendications précédentes, **caractérisé en ce que** les réseaux de diffraction à déphasage (G1, G2) sont inclinés conjointement ou l'un par rapport à l'autre le long de l'axe optique pour ajuster les déphasages des réseaux de diffraction à déphasage (G1, G2) pour qu'ils soient de déphasage $\pi$ ou de déphasage $\pi/2$ afin de correspondre à une énergie de photon ajustable permettant à l'agencement de fonctionner à une énergie de photon différente avec les mêmes réseaux de diffraction à déphasage.

8. Agencement selon l'une quelconque des revendications précédentes, l'échantillon (S) à analyser étant placé entre la source de rayons X ou le réseau de diffraction par absorption (G0) si le réseau de diffraction par absorption (G0) est utilisé et le premier réseau de diffraction à déphasage (G1), ou entre le premier réseau de diffraction à déphasage (G1) et le second réseau de diffraction à déphasage (G2), ou entre le second réseau de diffraction à déphasage (G2) et le détecteur.

9. Agencement selon l'une quelconque des revendications précédentes, **caractérisé en ce que** l'un des réseaux de diffraction (G0, G1 et G2) est latéralement déplacé avec une fraction de son pas et enregistre une série d'images sur le détecteur en termes de balayage par décalage de phase, et une procédure d'analyse est mise en œuvre pour les données de ce balayage par décalage de phase qui comprend les étapes de calcul, pour chaque pixel du détecteur, de la transformée de Fourier de la courbe d'intensité mesurée dans le pixel, et ensuite le maintien des composantes de Fourier comme signaux pour un traitement ultérieur.

10. Agencement selon l'une quelconque des revendications précédentes, les réseaux de diffraction à déphasage (G1, G2) étant fabriqués sur les deux côtés d'une galette, ayant des structures de réseau de diffraction soit parallèles l'une à l'autre soit ayant un angle prédéfini.

11. Agencement selon l'une quelconque des revendications précédentes, le premier réseau de diffraction à déphasage (G1) étant un réseau de diffraction 2D de type damier/maille, et le second réseau de diffraction (G2) étant un réseau de diffraction à réseau 2D, avec chaque élément du réseau étant un réseau de diffraction circulaire.

12. Agencement selon l'une quelconque des revendications précédentes, les deux réseaux de diffraction à déphasage (G1, G2) étant des réseaux de diffraction

par absorption.

FIG 1

detector

G2

G1

sample

source   G0

z

y

x

## FIG 2A

## FIG 2B

Virtual structured illumination (G1') generated by G1

Interference pattern (G2') on the detector plane

## FIG 2C

## FIG 3A

## FIG 3B

## FIG 3C

## FIG 3D

## FIG 4

Mean Visibility [%]

12.4 ▬▬▬▬▬▬▬▬▬▬▬▬▬▬▬▬▬▬▬▬▬▬▬▬▬▬▬▬▬▬▬ 24.63

## FIG 5A

1cm

## FIG 5B

1cm

## FIG 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015036795 A
- WO 2014194995 A

- EP 1447046 A1, C. David
- US 2015036795 A1, E. Roessl and T. Koehler